# EUROPEAN PATENT APPLICATION

(11) **EP 1 905 429 A1**
(43) Date of publication of application: **02.04.2008**
(21) Application number: 06291458.5
(22) Date of filing: 18.09.2006
(51) Int. Cl.: A61K 9/20, A61K 9/50

(54) **Orally dissolvable/disintegrable lyophilized dosage forms containing protected particles**

(71) Applicant: CEPHALON FRANCE, 94704 Maisons-Alfort Cedex (FR); Cima Labs, Inc., Eden Prairie, Minnesota 55344-9361 (US)
(72) Inventor: Khankari, Rajendra, Maple Grove, MN 55331 (US); Moe, Derek, Maple Grove, MN 55311 (US); Hamed, Ehab, Maple Grove, MN 55311 (US); Nguyen, Tam, 94450 Limeil-Brevannes (FR)
(74) Representative: Colombet, Alain André

(57) **Abstract**

The present invention provides an orally dissolvable/ disintegrable, lyophilized, dosage form adapted for direct oral dosing, including an active pharmaceutical ingredient ("API") containing particle which is coated with a lyophilizing solvent protective coating, which protects the form and/or attributes of the particle and/or API, and a matrix. There is also provided a method of making an orally dissolvable/disintegrable lyophilized dosage form in accordance with the present invention, and a method of treating a patient with a dosage form in accordance with the present invention.

## Description

### BACKGROUND OF THE INVENTION

Lyophilized dosage forms are known. See Lafon, U.S. Patent No. 4,616,047, Nguyen et al., U.S. Patent No. 5,843,347 and Blonde et al., U.S. Patent No. 3,855,712. These lyophilized dosage forms can be based on lyophilized emulsions such as that described in the '047 patent, and these formulations may include natural or synthetic sweetening agents such as saccharose, glucose, xylose, sorbitol, saccharine and saccharinates (especially the sodium, potassium and calcium saccharinates), the cyclamates (especially the sodium, potassium and calcium cyclamates), aspartame and taste modifying agents (in particular for the purpose of concealing the bitter after-taste of synthetic sweeteners of the saccharine and cyclamate type) such as in particular the citric, ascorbic and tartaric acids and aromas. The '347 patent describes, amongst other things, lyophilized microbeads which may then be coated with a host of compounds including polyacrylics and polymethacrylic acid esters ethyl/vinyl acetate copolymers, polymethylcyloxanes, polyacrylamides, PVP and polyvinyl acetate, polylactic and polyglycolic acids and copolymers thereof, polyurethanes, polypeptides and others. In one embodiment, the coating produces a microporous semipermeable membrane allowing the contained material to dissolve wherein an osmotic pressure is created which expels the aqueous solution containing the active ingredient. These formulations can include various diluents, sweeteners and artificial sweeteners, as well as natural or synthetic flavorings and mixtures thereof. See also U.S. Patent Nos. 4,490,407 and 3,939,260.

In non-lyophilized formulations, it is known to use various coating materials for taste masking. See U.S. Patent Nos. 5,178,878, 5,223,264, 6,024,981, and 6,740,341, all of which are assigned to CIMA LABS INC. of Eden Prairie, Minnesota.

However, a problem exists when using taste masking coating technology in the preparation of lyophilized dosage forms. Often the ingredients contained in a lyophilized dosage form are suspended, dispersed or even dissolved in lyophilization solvents which can include, for example, water, short chain normal alcohols, and the like. The lyophilization process can extend over a number of hours so solvent exposure can be prolonged. Sometimes these ingredients can be adversely affected by such solvent exposure, particularly prolonged exposure. Indeed, materials could unintentionally dissolve, thereby destroying some desirable attribute. A dissolved granulate, for example, is no longer a granulate. Similarly, with taste masked dosage forms, the taste masking coating could be compromised and thus the taste masking as well. Thus, there still remains a significant need for improvements in technology of producing dosage forms.

### SUMMARY OF THE INVENTION

The present invention provides, in one embodiment, an orally dissolvable/disintegrable, lyophilized, dosage form including an active pharmaceutical ingredient ("API") containing particle which is coated with a lyophilizing solvent protective coating, which protects the form and/or attributes of the particle and/or API. The term "protects the form and/or attributes" means preserving particles and/or the API unchanged, in the desired form or structure that the particles had prior to being contacted with lyophilization solvent. This includes, without limitation, preserving particle size, crystalline or amorphous form, degree of solvation or hydration, structure, color or fragrance and the like. The dosage forms of the present invention are intended for direct oral dosing which means that they will be placed into the mouth as a solid to be dissolved/disintegrated in the mouth and thereafter the resulting solution, dispersion, suspension or slurry is swallowed.

In one embodiment, taste masking can be accomplished through the use of a single taste masking layer which both protects the API particle from the lyophilization solvent and provides taste masking. In another embodiment, protection is obtained by overcoating a taste masking coated particle with second coating material which imparts solvent protection. Thus the term "protects the form and/or attributes" is also meant to include protecting coated particles to protect the integrity, structure and/or function of the coating. For another example, a controlled release coated particle, such as an enteric coated particle, when exposed to the lyophilization solvent, could be compromised. The API might, therefore, be released prematurely as in the mouth or stomach. Similarly, an extended release coating coated particle might be engineered to provide a zero order release, e.g. a constant release, over 12 hours or more. However, the solvent could compromise some of the coating whereby an unintended initial burst is realized and the release of the remaining API occurs in less than 12 hours. The invention is intended, in some embodiments, to provide protected particles that will not suffer from these problems because the API and coatings are not affected by solvent exposure.

The present invention is also directed to orally dissolvable/disintegrable, lyophilized dosage forms adapted for direct oral dosing. These dosage forms may comprise a particle coated with at least one lyophilizing solvent protective ("LSP") coating with the balance of the dosage form being a matrix. Particularly preferred are lyophilized dosage forms of the invention which employ taste masking coated particles or particles with a controlled or extended release.

In one embodiment, the dosage form comprises a particle of at least one active pharmaceutical ingredient ("API") coated with an LSP coating. This is also referred to herein as a "protected particle." It is preferred that the active ingredient be selected such that it will be substantially protected by the LSP coating. More preferably, the LSP coating is selected to be used in conjunction with an active that is substantially insoluble in the selected lyophilization solvent. The protected particle may also include other coating materials, solid supports, granulation binders, pH adjusting substances or buffers, and other excipients as described herein.

The dosage forms also include a matrix which may comprise one or more of a binder, filler, sugar, coating, flavor, polymer, a second active ingredient, pH adjusting substance, coloring agent, lubricant, effervescent disintegrant, noneffervescent disintegrant, viscosity modifier, surfactant, and buffer. This matrix is usually lyophilized as well, often along with the protected particles.

In the dosage forms of the invention, the particles coated with at least one LSP coating are generally present in an amount sufficient to provide a therapeutically effective amount of the API, with the balance being the matrix. Generally, the amount of API is about 0.01mg to about 2g by weight of the dosage form. In one particularly preferred embodiment, the dosage form comprises modafinil, famotidine or fentanyl and/or salts thereof as the API.

Processes of making the dosage forms of the invention allow for the inclusion of active ingredients and forms of active ingredients which were not previously possible as well as the realization of lyophilized dosage forms never previously thought possible. These processes are also considered to be a part of the invention.

Thus, in another embodiment of the present invention, there is provided a method of making an orally dissolvable/disintegrable lyophilized dosage form comprising the steps of providing at least one API, or a particle or coated particle including an API, coating same with an LSP coating (or plurality of LSP coatings), mixing the resulting protected particles with a matrix, and lyophilizing same to form a dosage form.

In yet another embodiment of the present invention, there is provided a method of treating a patient using any orally dissolvable/disintegrable lyophilized dosage form described herein comprising the steps of placing the dosage form in the mouth of a patient in need of treatment, allowing the dosage form to disintegrate/dissolve sufficiently to allow it, and in particular, the protected particles, to be swallowed as a solution, suspension or slurry, and allowing the patient to swallow the at least partially disintegrated/dissolved dosage form. This method is particularly useful for persons who cannot or will not swallow, geriatric patients, mental patients, and children.

In one embodiment, there is provided a dosage form comprising: at least one particle comprising an active pharmaceutical ingredient coated with a lyophilizing solvent protective coating which protects the form and/or attributes of said particle when in contact with a lyophilization solvent, and a matrix, said dosage form being a lyophilized, orally dissolvable/disintegrable dosage form adapted for direct oral dosing in the mouth of a patient. In one embodiment, another coating may be disposed between said particle and said lyophilizing solvent protective coating.

The present invention also relates to a method of making a dosage form comprising the steps of: providing at least one active pharmaceutically ingredient containing particle or coated active pharmaceutically ingredient containing particle, coating same with at least one lyophilizing solvent protective coating, forming a mixture of said lyophilizing solvent protective coated particles with a lyophilizing solvent which will not dissolve said lyophilizing solvent protective coating, and lyophilizing said mixture to form a dosage form. In one embodiment, the mixture also includes the matrix. In another embodiment, the method also includes the step of placing said mixture into a portion of a container prior to lyophilzation and sealing said mixture into said dosage form after lyophilization.

### DETAILED DESCRIPTION

The "dosage form(s) adapted for direct oral dosing" or more simply, the "dosage form(s)" of the invention are defined as solids such as tablets, capsule, caplets, wafers, films and the like adapted to rapidly dissolve/disintegrate in the mouth. Most preferred are tablets, caplets and wafers. After the dosage form dissolves and/or disintegrates in the mouth, the resulting solution, slurry or suspension (which includes the protected particles) is swallowed such that the active ingredient can enter the digestive tract down stream of the mouth. The active ingredient then enters the blood stream via, for example, the stomach and/or intestines. These dosage forms generally comprise API containing protected particles and a matrix.

"Orally dissolve(able)/disintegrate(able)" means that the water soluble ingredients within the dosage form will dissolve and/or disintegrate sufficiently to allow ingestion as a solution, suspension or slurry of, *inter alia*, protected particles. Most preferably, the solution, suspension or slurry will be considered generally non-gritty, and will be pleasant in terms of viscosity and mouth feel by taste tests according to those of skill in the art.

It should be understood that whether a dosage form in accordance with the invention can be considered "dissolvable" or "disintegrable" or both depends upon, at least, the nature of the dosage form, the load of active ingredient, the solubility of the active ingredient, the size of the dosage form and the materials used. "Dissolving" in accordance with the present invention is a process similar to melting in one's mouth and depends upon a number of factors, most importantly, the degree of water solubility of the ingredients in question and how much of the dosage form is composed of highly water-soluble ingredients. This involves the nature of the material (its inherent solubility in water at 25 degrees C. (in a glass) or 37 degrees C. (in the mouth)) as well as things like particle size, average particle size, porosity, form (crystalline, amorphous, solid solution) and the like. However, it will be appreciated that a material that is inherently not water soluble will not suddenly become water soluble merely by, for example, reducing its average particle size. "Disintegration" and like terms mean that the dosage form falls apart into smaller particles and/or aggregates. As will be appreciated, when a dosage form in accordance with the present invention is placed in the mouth, and preferably on the tongue, portions of the dosage form will break down and others will begin to dissolve in the period between being placed in the mouth and being swallowed. Indeed, some materials will both break apart and begin to dissolve. Unless the context suggests otherwise the use of either "dissolve" or "disintegrate" is meant to imply the possibility of either or both processes.

The dosage forms of the invention preferably will rapidly dissolve and/or disintegrate sufficiently in the mouth such as, for example, within 120 seconds or less, preferably 60 seconds or less, more preferably 40 seconds or less, and even more preferably 30 seconds or less, so as to allow them to be swallowed as a solution, suspension or a slurry containing the protected particles. In some embodiments, disintegration/dissolution occurs in about 10 seconds or less. Preferably, the dosage forms also provide a pleasant organoleptic sensation. Generally, if the dosage form will disintegrate in accordance with the USP disintegration test within 60 seconds or less, it is likely to meet the in-mouth dissolution/disintegration standards described herein. See 29/24 USP/NF 2671. This test is used to determine whether dosage forms disintegrate within a prescribed time when placed in a liquid medium under certain conditions.

"Solvent insoluble" in accordance with the present invention means that insoluble ingredients within the protected particle and/or the dosage form will not readily dissolve or disintegrate sufficiently when contacted with a lyophilization solvent for a period of between 1 and 6 hours, and more preferably a period of between 2 and 4 hours. This can be measured by centrifuging the dispersion to separate the particles from the solution followed by measuring solution concentration. While it is preferred that the LSP coating and active ingredient be selected based on their solvent insolubility, it will be appreciated that such selection depends upon a number of factors, including, but not limited to, the taste of the active ingredient, the amount of active ingredient and/or LSP coating, the ratio of active ingredient to LSP coating, the degree of taste masking imparted to the dosage form by the matrix, or other ingredients added to the LSP coating, other coatings added to the dosage form, etc. Thus, it is contemplated by this invention that the active ingredient(s), and/or other ingredients within the dosage form may be solvent insoluble, partially solvent insoluble, or solvent soluble. It is also noted that thicker LSP coatings will impart longer insolubility when contacted with a lyophilization solvent. In addition, it is also contemplated by this invention that the lyophilization solvent may be selected to reduce solubility of the LSP coating, active ingredient, and/or the dosage form, including but not limited to controlling pH levels and ionic strength of the lyophilization solvent to help prevent leakage or change.

"Controlled release" in accordance with the present invention means a release of the active pharmaceutical ingredient ("API") from the protected particles at a particular desired point in time once the dosage form has been placed into the mouth of a patient. Generally, it involves a delayed but otherwise complete release. A sudden and total release in the stomach at a desired and appointed time or a release in the intestines such as through the use of an enteric coating, are both considered controlled release, as opposed to a passive release dosage form wherein within moments of reaching the stomach, the API begins to be exposed to digestion and possibly the absorption process (such as occurs when swallowing a raw powdered API). Controlled release requires that release occur at a predetermined time or in a predetermined place within the digestive tract. It is not meant to be a passive, uncontrolled process as in swallowing a normal tablet.

An "extended release" dosage form is a dosage form (or particles) which generally begins its release and continues that release over an extended period of time. Release can occur beginning almost immediately or can be delayed, in which case the extended release dosage form is also a controlled release dosage form as described above. Release can be constant, can increase or decrease over time, can be pulsed, can be continuous or intermittent, and the like. Generally, however, the release of the API from an extended release dosage form will exceed the amount of time of release of the drug taken as a normal, passive release tablet. Thus, for example, while all of the API of an uncoated aspirin tablet should be released within, for example, four hours, an extended release dosage form could release a smaller amount of aspirin over a period of six hours, 12 hours, or even longer. Extended release in accordance with the present invention generally means that the release occurs for a period of six hours or more, and more preferably 12 hours or more.

"Particle" herein is used to encompass any API in any form. This can include, without limitation: grains, microparticles, crystals, granules (wet or dry granulation), microgranules, agglomerates, pellets, mini-tablets, beads, solid supports, microcrystals and powders, as well as coated versions of any of the foregoing. For example, a particle could be a crystal of a particular API. A particle could also be an enterically coated crystal of that same API. In still another embodiment, the particle could be an API mixed with excipients such as a swelling agent which, when wetted, gels and provides extended release. Similarly, a particle could be a sugar sphere coated on its surface with an API or API containing layer. For purposes of the present invention, the size of the particle generally does not matter.

"Lyophilizing solvent protective coating" or "LSP coating" in accordance with the present invention means a coating applied to a particle as defined above (whether already coated or not) which provides protection thereto from lyophilization solvents. This LSP coating is intended to maintain the form and/or attributes of the particle and/or coating, or at least slow the release and/or extent of such API particles being changed or degraded. In particular, it is meant to stop the unintended and undesired leakage of the API from the dosage form and/or the particle. In a most preferred aspect, but one which is not necessary for the functioning of the invention, the LSP coating should preserve at least one of the structural integrity, function and/or physical attributes of the API, the particle and/or its coating during the lyophilization process and thereafter. These LSP coatings may themselves also provide taste masking, provide controlled or extended release, or may impart additional advantages. Most importantly, however, the LSP coating will provide protection for the particle during lyophilization but will impart little or no "unintended change" to the intended release of the API (instantaneous, controlled or extended) once a dosage form (or protected particles) containing same clears a patient's mouth.

There are generally two tests that are used to determine "leakage" of a dosage form. In the first, a microscope is used to evaluate the particle. The dosage form, or, as appropriate, the particle, is placed into a solution for a length of time, generally 2 to 4 hours, then removed from the solution and inspected under a microscope. While this test will show swelling, and general changes to the exterior of the dosage form, it is not an accurate detection of leakage and is therefore not the recommended test. The second, quantitative test requires placing the dosage form, or, as appropriate, the particle, into a solution for a length of time, again generally 2 to 4 hours, centrifuging the sample, removing the supernatant and quantitatively measuring for the amount of active ingredient which may have been released. The second test is more accurate in determining any change to the dosage form, including any release of the active ingredient. Thus, for the present invention, the second, quantitative test is the preferred method of determining leakage.

In a preferred embodiment of the present invention, the amount of leakage, as measured by the quantitative test, is preferably less than 20%, and more preferably less than 10%, and most preferably less than 5%, depending on the solubility of the active ingredient and the amount of LSP coating. In accordance with the present invention, when the active ingredient is solvent insoluble, it is preferred that the leakage is less than 10% and more preferably less than 5%. It is also contemplated by the present invention that when a solvent soluble active ingredient is used, an increase in the amount of LSP coating, and/or control of pH and/or ionic strength of the solvent may also be manipulated to reduce leakage and unintended change.

If the LSP coated particle was subcoated with an enteric protecting material which also could provide taste masking, then there is no need for the LSP coating to remain intact or functional following the completion of lyophilization. Accordingly, such coating could begin dissolving even while the protected particles are in the mouth. It should not provide any impediment to the eventual functioning of the enteric coating once the protected particles reach the intestines. Any particle defined herein coated in an LSP coating is a "protected particle" in accordance herewith. Note that in the context of protected particles, the extended release, controlled release, taste masked particles, for example are "coated" with an LSP coating. Thus, the extended release, controlled release or taste masking material is disposed "under the LSP coating. However in this context, "coating" should be interpreted broadly to include any material mixed, granulated, agglomerated or coated with the active to produce a particle. For example, some extended release dosage forms use a material that swells when wetted and this prevents rapid release of the active. These materials may be coated onto the active or mixed therewith. In either event, they are disposed "under" the LSP coating. For convenience, these extended release, controlled release and taste masking materials will be generically referred to as a "coating" disposed under the LSP coating.

"Pleasant organoleptic sensation" is used here to mean that the oral dosage form of some embodiments does not provide a relatively gritty sensation, has a good mouth feel in terms of viscosity, cohesion of the particulate and the like and provides adequate taste masking so as to make ingestion palatable and preferably pleasing.

"Solution, slurry, or suspension" means that the dosage form is mixed with an adequate amount of liquid (water/saliva) so that the dosage form dissolves/disintegrates adequately to create a solution or a liquid containing suspended solids, and in particular, the protected particles which can easily be swallowed.

"Effervescent" means that the dosage form, when mixed with liquid, including water and saliva, will evolve a gas. Preferred effervescent agents (or effervescent couple) evolve gas by means of a chemical reaction which takes place upon exposure of the effervescent disintegration agent to water and/or to saliva in the mouth. This reaction is most often the result of the reaction of a soluble acid source and an alkali monocarbonate or carbonate source. The reaction of these two general compounds produces carbon dioxide gas upon contact with water or saliva. Such water-activated materials must be kept in a generally anhydrous state and with little or no absorbed moisture or in a stable hydrated form, since exposure to water will prematurely disintegrate the tablet. Of course, an effervescent couple (or the individual acid and base separately) can be coated with an LSP coating to prevent premature reaction. Such a couple can also be mixed with previously lyophilized particles coated with an LSP coating as described herein.

The dosage forms of the present invention include a particle coated with an LSP coating and a matrix. The particle comprises at least one first API, although it can include other APIs or any other pharmaceutical excipients as well. These can include solid supports, beads, binders, disintegrants, pH adjusting substances, buffers and the like. The particles are preferably present in one or more dosage forms in an amount sufficient to provide a therapeutically effective amount of said at least one API.

A "therapeutically effective amount" is the amount or quantity of a drug or API which is sufficient to elicit the required or desired therapeutic response, or in other words, the amount which is sufficient to elicit an appreciable biological response when administered to a patient. The does need not be optimal, nor even provide a cure or symptomatic relief. As used with reference to a vitamin or mineral, the term "effective amount" means an amount at least about 10% of the United States Recommended Daily Allowance ("RDA") of that particular ingredient for a patient. For example, if an intended ingredient is vitamin C, then an effective amount of vitamin C would include an amount of vitamin C sufficient to provide 10% or more of the RDA.

APIs, also referred to herein as "drugs" or "active ingredients," in accordance with the present invention, include materials capable of being lyophilized, or otherwise useful in the present invention. Such APIs may include pharmaceutical ingredients, vitamins, minerals and dietary supplements. A combination or mixture of any of the foregoing is also contemplated by the present invention. Pharmaceutical ingredients may include, without limitation, antacids, analgesics, anti-inflammatory, antibiotics, diuretics, anorexics, antihistamines, antiasthmatics, antidiuretics, antiflatuents, antimigraine agents, antispasmodics, sedatives, antihyperactives, antihypertensives, tranquilizers, decongestants, immunosuppressants, anticancers, antivirals, antiparasitics, antifungals, antiemetics, antidepressants, antiepileptics, local anesthetics, vasoactive agents, skeletal muscle relaxants, drugs for parkinsonism, antipsychotics, hematopoietic growth factors, antihyperlipidemics, anticoagulants, fibrinolytics, antithrombotics, hormones, therapeutic proteins and peptides, antiarrhythmia, antiangina, beta blockers and combinations thereof. In one embodiment in accordance with the present invention, the API is fentanyl or modafinil or salts thereof.

As used in this disclosure, the term "vitamin" refers to trace organic substances that are required in the diet. For the purposes of the present invention, vitamin(s) include, without limitation, thiamin, riboflavin, nicotinic acid, pantothenic acid, pyridoxines, biotin, folic acid, vitamin B12, lipoic acid, ascorbic acid, vitamin A, vitamin D, vitamin E and vitamin K. Also included within the term vitamin are the coenzymes thereof. Coenzymes are specific chemical forms of vitamins. Coenzymes that may be useful in the present invention include thiamine pyrophosphates (TPP), flavin mononucleotide (FMM), flavin adenine dinucleotive (FAD), Nicotinamide adenine dinucleotide (AND), Nicotinamide adenine dinucleotide phosphate (NADP) Coenzyme A (CoA) pyridoxal phosphate, biocytin, tetrahydrofolic acid, coenzyme B₁₂, lipoyllysine, 11-cis-retinal, and 1,25-dihydroxycholecalciferol. The term vitamin(s) also includes choline, carnitine, and alpha, beta, and gamma carotenes.

As used in this disclosure, the term "mineral" refers to inorganic substances, metals, and the like required in the human diet. Thus, the term "mineral" as used herein includes, without limitation, calcium, iron, zinc, selenium, copper, iodine, magnesium, phosphorus, chromium and the like, and mixtures thereof.

The term "dietary supplement" as used herein means a substance which has an appreciable nutritional effect when administered in small amounts. Dietary supplements include, without limitation, such ingredients as bee pollen, bran, wheat germ, kelp, cod liver oil, ginseng, and fish oils, amino-acids, proteins and mixtures thereof. As will be appreciated, dietary supplements may incorporate vitamins and minerals.

While at least one API is desired, multiple APIs may also be used and found in a single or in multiple particles (e.g. two API granulated together or in separate particles). So too the dosage form may include lyophilized and nonlyophilized APIs. For example, a lyophilized material or tablet could be mixed with nonlyophilized material and both housed in a single gelatin capsule or a lyophilized dosage form could be coated with a material which includes an API.

The amount of API used can vary greatly and can depend upon, among other things, the type and properties of the API, the condition it is intended to treat, the size and type of the patient, the size, nature and number of the dosage form(s), whether or not more than one API is to be delivered from the dosage form and how many dosage forms will be used to deliver each dose. Generally, the total amount of API for any individual dosage form is a therapeutically effective amount, and in certain embodiments, an amount of about 0.01 mg to about 2.0g by weight, more preferably about 0.05mg to about 1g, and most preferably about 1mg to about 800mg. This is based on the amount of API only -- not counting, for example, the amount of LSP coating, and/or any other coating or excipient in the final particle. In terms of the proportion of the protected particles that are APIs, the API can range from about 0.1 % to about 99.9% by weight of the protected particle, and more preferably in an amount of about 50% to about 98% by weight thereof, and most preferably in an amount of about 80% to about 95% by weight thereof. The balance would be any coating(s), LSP coating(s) and excipients.

The LSP coating that is used to protect particles in accordance with the present invention from dissolution or degradation while in contact with lyophilizing solvents often makes up the balance of the weight percentage of the protected particles. In other instances, the balance includes other coatings and/or excipients such as binders and the like.

The LSP coating generally is made from any natural or synthetic polymer including: acrylic polymers, modified celluloses, and the like, which are pH dependant materials. In one embodiment, the coating materials will become soluble at a pH which is generally acidic (pH 7 or below) and in another embodiment the coating materials will become soluble at a pH which is generally basic (pH 7 or above). In a third embodiment, the materials become soluble at a generally neutral pH (pH 6-8). There are a number of factors which determine which material is used as an LSP coating in a given situation including, without limit, ease of handling and processing, cost, thickness, site of intended dissolution of the LSP coating and/or the API, and the type and pH of the lyophilization solvent to be used.

In one preferred embodiment, the LSP coating is made from a material that becomes soluble at a pH of about 6.5 or below. In another embodiment, the coating becomes soluble at a pH of between about 6.0 and about 6.5. These polymers and copolymers should preferably be pharmacologically acceptable, capable of providing appropriate release and while still being convenient to process. These include, for example, acrylic polymers, modified celluloses amino alkyl acrylate copolymers such as, for example, copolymers of methylmethacrylate, butylmethacrylate and dimethylaminoethyl methacrylate. See European Pharmacopoeia 4.4 (04/2003:1975) at 3385. In one particularly preferred embodiment, the copolymer has a relative molecular mass of about 150,000 and a ratio of dimethylaminoethyl methacrylate groups to butylmethacrylate groups and methylmethacrylate groups of about 2:1:1 and the content of the dimethylaminoethyl groups is about 20.8% to 25.5% based on the amount of dry substances present.

A particularly preferred material can be obtained under the mark Eudragit E-100, which can be used in normal form or in micronized Eudragit E-100 and mixtures thereof. Eudragit is a trademark of Rohm GmbH, Chemische Fabrik, Kirschenallee, D-64293, Darmstadt, Germany for a group of acrylic polymers.

These materials are generally solid at room temperature. However, they may be applied by being dissolved, suspended, emulsified, dispersed or the like in a solvent or solvent system. Preferred solvents in accordance with the present invention include those capable of substantially dissolving or dispersing Eudragit E-100 such as water, normal C₁-C₅ alcohol, branched C₁-C₅ alcohol, denatured C₁-C₅ alcohol, and low molecular weight ketones such as acetone and MEK. Ethanols, including (SDA-3A) and denatured ethanol are most preferred.

In one embodiment, the total amount of LSP coating on the particle ranges from about 0.1 % to about 500% by weight of the coated particle. (in these instances, "particles" include the weight of the API and any other taste masking, controlled release or delayed release coatings and any excipients and is measured as percent weight gain based on a finite sample of particles.) And more preferably in an amount of about 1 % to about 200% by weight of the particle. Most preferably in an amount of about 1% to about 100% by weight. Of course, this is measured as a percent weight gain and is dependent upon the kind(s) of API used, the kind or type of coating(s) used, the amount of API to be delivered in the dose, etc.

In one embodiment in accordance with the present invention, these particles are "fully" coated with an LSP coating. The use of a "full" coating means an amount of coating which is sufficient to ensure that substantially all of the particles are completely coated. This will provide nearly complete taste masking and/or nearly complete absence of any dissolution from or change in the coated particles. Thus, for a "full" coating, the amount of LSP coating material is an amount sufficient to increase the weight of the particles by at least about 5%, more preferably at least about 10% and most preferably at least about 20%.

The dosage form of the invention also includes a matrix, making up the balance of the dosage form. The matrix comprises one or more of a binder, lyophilization binder, filler, sugar, artificial sweetener, polymer, flavoring agent, taste masking material, active ingredient, coloring agent, lubricant, effervescent disintegrant, noneffervescent disintegrant, viscosity modifier, surfactant and buffer. Any conventional material may be used to provide a matrix in accordance with the present invention, so long as it meets the overall objectives of the present invention, including dissolvability/disintegratability in the mouth. The amount of any one or more of these ingredients will vary with the amount of API, protected particle size, and degree of coating, shape of the dosage form, rate of dissolution/disintegration, the need for complimentary taste masking, how many ingredients are used, which ingredients are used, etc. Any combination or amounts are contemplated sufficient to allow the creation of a soluble, storable dosage form in accordance with the present invention, particularly one exhibiting a pleasant organoleptic sensation.

Binders can be anything known to be used as binders. Some binders that may be useful in the present invention include acacia, tragacanth, gelatin, starch, cellulosic materials such as methyl cellulose, sodium carboxymethylcellulose, hydroxypropylmethylcellulose, hydroxypropylcellulose, hydroxyethylcellulose, polyvinyl pyrrolidone, alginic acids and salts thereof, magnesium aluminum silicate, polyethylene glycol, guar gum, polysaccharide acids, bentonites, sugars, invert sugars, xanthan gum, Dextran 20, 40 70, povidone, copovidone, cyclodextrines and derivatives, and the like. Binders can be used in conventional amounts and preferably in an amount of about 0.2% by weight to about 20% and more preferably about 2% to about 10% percent by weight of the total dosage form.

Lyophilization binders can be anything known to be used as a lyophilization binder. Some lyophilization binders that may be useful in the present invention include Dextran 70, povidone, and methyl cellulose. Lyophilization binders can be used in conventional amounts and preferably in an amount of about 2% to about 10%, and more preferably from about 4% to about 8%.

Fillers can be anything known to be used as fillers. Some fillers that may be useful in the present invention include mannitol, dextrose, sorbitol, lactose, sucrose, and calcium carbonate. Fillers can be used in conventional amounts and preferably in an amount of about 0.5% to about 99%, and more preferably about 5% to about 50%.

A particularly preferred type of filler which may be used is sugars. Sugars that may be used in the present invention include sugar, sugar alcohols, ketoses, saccharides, polysaccharides, oligosaccharides and the like, as well as celluloses and modified celluloses.

Sugars may also include direct compression and/or nondirect compression sugars. Particularly preferred nondirect compression sugars include, without limitation, dextrose, mannitol, sorbitol, trehalose, lactose and sucrose. Of course, these sugars generally exist as either a direct compression sugar, i.e., a sugar which has been modified to increase its compressibility and/or flow, or a nondirect compression sugar which does not have sufficient flowability and/or compressibility to allow it to be used in high speed processing and multi-tablet presses without some sort of augmentation such as, without limitation, a glidant to increase flow, granulation to increase flow and/or compressibility and the like. Of course, techniques like granulation can also be used to convert something which initially has sufficient flow and compressibility to be considered a direct compression sugar before processing into a nondirect compression sugar as well. This can be measured by directly compressing tablets made only from a sugar and comparing the flow and compressibility both before and after processing. If flow and/or compressibility are reduced after processing the material is likely to have become a nondirect compression sugar. It will be appreciated however, that whether or not the reduction in properties are sufficient to require augmentation or further processing before the sugar is used in a commercial process will depend on a number of factors including the amount used, the type of processing equipment used, and the overall formulation. Generally, however, some further processing or augmentation is required. While not definitive, sometimes a nondirect compression sugar will have at least about 90% of its particles smaller than about 200 microns, and more preferably 80% smaller than about 150 microns.

The amount of total sugar can range from about 0.5% to about 95%. More preferably, the amount of sugar will range from about 5% to about 50%, and even more preferably between about 5% and 25%.

Artificial sweeteners can be anything known to be used as artificial sweeteners. Some artificial sweeteners that may be useful in the present invention without limitation include saccharin, aspartame, sucralose, neotame, and acesulfame potassium. Artificial sweeteners may be used in conventional amounts, and preferably in an amount ranging from about 0.1 % to about 10%.

Polymers may include natural or synthetic polymer including acrylic polymers, modified celluloses, and the like. In one embodiment, the coating materials are pH dependant materials that become soluble at a pH of 6.0-6.5 or below such as EUDRAGIT, from Rohn & GmbH, Chemische Fabrik, Kirschenallee, D-64293 Darmstadt, Germany, a group of acrylic polymers including for example, without limitation EUDRAGIT E100, modified celluloses such as ethyl cellulose, methyl cellulose, hydroxypropylcellulose, sodium carboxy methyl cellulose, hydroxyethylcellulose, poloxmer, and/or hydroxypropylmethylcellulose. Polymers may be used in conventional amounts, and preferably in an amount ranging from about 0% up to about 15%.

Flavoring agents can be anything known to be used as flavoring agents. Flavoring agents that may be useful in the present invention may include synthetic flavor oils and flavoring aromatics and/or natural oils, extracts from plants, leaves, flowers, fruits and so forth and combinations thereof. These may include cinnamon oil, oil of wintergreen, peppermint oils, clove oil, bay oil, anise oil, eucalyptus, thyme oil, cedar leave oil, oil of nutmeg, oil of sage, oil of bitter almonds and cassia oil. Also useful as flavoring agents are vanilla, citrus oil, including lemon, orange, banana, grape, lime and grapefruit, and fruit essences, including apple, pear, peach, strawberry, raspberry, cherry, plum, pineapple, apricot and so forth.

Flavoring agents may be used in conventional amounts, and preferably in an amount ranging from about 0% to about 20% by weight of the dosage form, and more preferably from about 1% to about 10% by weight of the dosage form, and most preferably from about 3% to about 8% by weight of the dosage form.

Coloring agents can be anything known to be used as a coloring agent. Coloring agents useful in the present invention may include titanium dioxide, and dyes suitable for food such as those known as F. D. & C. dyes and natural coloring agents such as grape skin extract, beet red powder, beta-carotene, annatto, carmine, turmeric, paprika, etc. Coloring may be used in conventional amounts, and preferably in an amount ranging from about 0% to about 5% by weight of the dosage form.

Lubricants can be anything known to be used as a lubricant. Lubricants that may be useful in the present invention may include intrinsic or extrinsic lubricants. Intrinsic lubricants may include magnesium, calcium, zinc salts of stearic acid, hydrogenated and partially hydrogenated vegetable oils, animal fats, polyethylene glycol, polyoxyethylene monostearate, talc, light mineral oils, sodium benzoate, sodium lauryl sulphate, magnesium oxide and the like. Lubricants may be used in conventional amounts, and preferably in an amount from about 0% to about 5% by weight of the dosage form.

Disintegrants useful in the present invention may be effervescent or noneffervescent. Effervescent disintegration agents useful in the present invention can be anything known to be used as an effervescent disintegration, such as described in Wehling et al., U.S. Patent No. 5,178,878 cols. 5-7, incorporated by reference herein. The acid sources or acid for the effervescent agent may be any which are safe for human consumption and may generally include food acids, acid anhydrides and acid salts. Food acids include citric acid, tartaric acid, malic acid, fumaric acid, adipic acid, and succinic acids etc. Because these acids are directly ingested, their overall solubility in water is less important than it would be if the effervescent tablet formulations of the present invention were intended to be dissolved in a glass of water. Acid anhydrides and acid of the above described acids may also be used. Acid salts may include sodium, dihydrogen phosphate, disodium dihydrogen pyrophosphate, acid citrate salts and sodium acid sulfite.

Carbonate sources include dry solid carbonate and bicarbonate salts such as sodium bicarbonate, sodium carbonate, potassium bicarbonate and potassium carbonate, magnesium carbonate and sodium sesquicarbonate, sodium glycine carbonate, L-lysine carbonate, arginine carbonate and amorphous calcium carbonate.

The effervescent disintegration agent(s) of the present invention is not always based upon a reaction which forms carbon dioxide. Reactants which evolve oxygen or other gasses which are pediatrically safe are also considered within the scope. Where the effervescent agent includes two mutually reactive components, such as an acid source and a carbonate source, it is preferred that both components react completely. Therefore, an equivalent ratio of components which provides for equal equivalents is preferred. For example, if the acid used is diprotic, then either twice the amount of a mono-reactive carbonate base, or an equal amount of a di-reactive base should be used for complete neutralization to be realized. However, in other embodiments of the present invention, the amount of either acid or carbonate source may exceed the amount of the other component. This may be useful to enhance taste and/or performance of a tablet containing an overage of either component. In this case, it is acceptable that the additional amount of either component may remain unreacted.

In general, the amount of effervescent disintegration agent of the present invention useful for the formation of a dosage form according to the present invention should range from about 0% to about 10% of the final composition.

Noneffervescent disintegrants can be anything known to be used as noneffervescent disintegrants. Some noneffervescent disintegrants that may be useful in the present invention include microcrystalline cellulose, (AVICEL PH 200, AVICEL PH 113, AVICEL PH 101), AC-Di-Sol (Croscaramellose Sodium) and PVP-XL (a crosslinked polyvinylpyrrolidone); a starch glycolate such as sodium starch glycolate; starches and modified starches; polymers; hydroxyalkyl cellulose such as hydroxymethylcellulose, hydroxypropylcellulose, and hydroxyopropylmethylcellulose, as well as compounds such as carbopol; sweeteners; clays, such as bentonite; alginates; gums such as agar, Arabic, xanthan, guar, locust bean, karaya, pectin and tragacanth. Noneffervescent disintegrants may be used in conventional amounts and preferably in an amount of about 0% to about 15% by weight of the dosage form.

Viscosity modifiers can be anything known to used as a viscosity modifier. Some viscosity modifiers that may be useful in the present invention include, without limitation, sodium alginate, hydroxypropyl methylcellulose (HPMC), hydroxyethylcellulose (HEC), sodium carboxymethycellulose (sodium CMC), polyvinylpyrrolidone (PVP), Konjac flour, carrageenan, xanthan gum, other hydrophilic polymers, or mixtures thereof. Viscosity modifiers can be used in conventional amounts and preferably in an amount of about 0% to about 5%, and more preferably in an amount of about 0.05% to about 0.5% by weight of the extra-granular matrix.

Surfactants can be anything known to be used as surfactants: anionic, cationic, amphoteric, nonionic surfactants such as sucroesters 7-11-15; polysorbates 20-60-80; poloxamers 188-407; sorbitan stearate, etc. Some surfactants that may be useful in the present invention include, without limitation, various grades of the following commercial products: Arlacel®, Tween®, Capmul®, Centrophase®., Cremophor®, Labrafac®, Labrafil®, Labrasol®, Myverol®, Tagat®, and any non-toxic short and medium chain alcohols. Surfactants can be used in conventional amounts and preferably in an amount of about 0% to about 10%, and more preferably in an amount of about 0.5% to about 5% by weight of the extra-granular matrix.

Buffers can be anything known to be used as a buffer. Some buffers that may be useful in the present invention include any weak acid or weak base or, preferably, any buffer system that is not harmful to the gastrointestinal mucosa. These include, but are not limited to, any of the acids or bases previously mentioned as the effervescent components, sodium carbonate, potassium carbonate, potassium carbonate, disodium hydrogen phosphate, sodium dihydrogen phosphate, and the equivalent potassium salts. Buffers can be used in conventional amounts and preferably in an amount of about 0.1 % to about 5%, and more preferably in an amount of about 0.2% to about 2% by weight of the matrix.

As long as a resultant dosage form meets the overall objectives of the present invention, and contains a therapeutically effective amount of at least one API, the amount of matrix is not critical. It is contemplated that the amount of matrix, and the ingredients comprising that matrix will vary.

However, generally the total amount of API containing particles coated to protect them during lyophilization for any individual dosage form is preferably from 0.1 % to about 90%, and more preferably from 1% to about 80% by weight of the dosage form.

Generally, dosage forms in accordance with the present invention can be produced by the steps of coating an API-containing particle with the LSP coating, mixing the resulting protected particle with the matrix and lyophilizing same into a desired form including tablet, wafer, caplet, etc. Coating may be accomplished in any known way. Coating can be accomplished using, for example, a Wurster fluidized bed where the coating material enters from the bottom of the reactor. See also the techniques described in U.S. Patent No. 6,024,981, 4,949,588, 5,178,878, and 5,055,306. See also Lieberman, Pharmaceutical Dosage Form: Tablets Vol. 1 (2d. ed. 1989) NY, 732-36. Other coating techniques contemplated by the present invention include spray drying, spray chilling, spray congealing, hot melt coating in fluid beds or through extrusion, fluid bed top spray mode, fluid bed tangential spray mode, conventional pan coating, perforated pan coating, microencapsulation through coacervation or other forms of phase separations, interfacial polymerization, super critical fluid coating, spinning disc coating and other centrifugation coating techniques.

The particles and, in some embodiments, the matrix are combined with a lyophilization solvent prior to lyophilization. Lyophilization solvents that may be useful in the present invention include, but are not limited to water, isopropyl alcohol, ethyl alcohol, and tertbutyl alcohol or combinations thereof. The solvent may also contain other ingredients such as flavors, sweeteners, polymers, binders, colorants, buffers and surfactants. The LSP coating should be selected such that it will not dissolve in the solvent, or should dissolve sufficiently slowly to provide substantial, if not complete protection, during exposure to lyophilization solvents. In one aspect of the present invention it is contemplated that some amount of solvent may be detected in the resulting dosage form, however it is preferred that the dosage form contain little if any residual solvent.

The blend of matrix materials, protected particles and solvent may be mixed for any period of time, under any conditions, using any kind of conventional blending apparatus to form a blend in accordance with this invention. Generally blending is done under conditions that would not alter the desirable properties of the coated particles or matrix materials, such as, without limitation, radically altering the particle size distribution. While it is generally desirable that the mixture be homogeneous, particularly where content uniformity is an issue, there might be instances where a random or patterned distribution is contemplated. Thus, blending could be more broadly thought of as any mixing operation.

The blend may then be poured into molds or shaped into a variety of forms for administration to a patient and lyophilized. Lyophilization is performed as described Lafon, U.S. Patent No. 4,616,047, Nguyen et al., U.S. Patent No. 5,843,347, and Blonde et al., U.S. Patent No. 3,855,712, all of which are herein incorporated by reference. Generally speaking, all of the raw materials, including the protected particles, are weighed and then a solution is prepared of the various soluble ingredients. A suspension of the protected particles and other insoluble materials, possibly with homogenization, is accomplished using a mixer or stirrer and then the suspension is poured into preformed blisters. These are then loaded into a lyophilizer wherein the materials are first frozen and then freeze dried. Finally, the blisters are sealed.

Consider the following mixture for illustration of a process in accordance with the invention.

**TABLE 1:**

| Ingredient | % | Unit formula |
|---|---|---|
| Protected particles | 77.00 % | 462.0 mg |
| Sucralose | 3.33 % | 20.0 mg |
| Dextran 70 | 4.16 % | 25.0 mg |
| Xanthan gum | 0.25% | 1.5 mg |
| Grape flavor | 5.00 % | 30.0 mg |
| Strawberry flavor | 3.33% | 20.0 mg |
| Disodium hydrogen phosphate | 0.50 % | 3.0 mg |
| Lactose monohydrate | 6.42 % | 38.5 mg |
| Purified water | | 700.0 mg |
| Total wet weight | | 1300.0 mg |
| Total unit dosage form weight | | 600.0 mg |

Using the formulation of Table 1 as a general guide, the xanthan gum and disodium hydrogen phosphate (Na₂HPO₄ anhydrous) are added to water and stirred at room temperature until obtaining a homogenous solution. The protected particles, sucralose, dextran 70, lactose, and flavor are introduced in a mixer equipped with a vacuum system and the powder blend is created by mixing at atmospheric pressure for up to about 15 minutes and then under low pressure (200-400 Hpa) for up to about the same amount of time. Then the solution of xanthan gum and disodium hydrogen phosphate is introduced into the mixer at low pressure (approximately 200 Hpa) and mixing continues for a few minutes. The wet suspension is mixed for about an hour at atmospheric pressure and room temperature and may optionally be mixed at alternating low and room temperatures and pressures thereafter until such time as a homogenous suspension is produced. The homogenous suspension is distributed into preformed blisters such as, for example, PVC, aluminum, and or ACLAR^{®} blisters. The unit weight and blister weight are monitored during filling. The blisters filled with the suspension are introduced into the freeze drier and frozen to a temperature below -25 degrees C. for at least about half an hour then freeze dried for upwards of four hours. The maximum temperature during most of the freeze drying operation is 60 degrees C. The blisters filled with the lyophilized product are then sealed with, for example, an aluminum foil in a controlled room of less than 40% relative humidity at a temperature of about 18-22 degrees C.

The hardness, disintegration time, and residual humidity are monitored. Without limitation, desirable products will have a hardness of about 10 Newtons or greater, and more preferably 15 Newtons or more, a friability of about 5% or less after 50 rotations in a standard friabulator and a disintegration time (based on an average of six tablets) in a standard USP disintegration apparatus, of less than 60 seconds. The solvent content is desirably less than 2%.

Once lyophilized, it is contemplated that the dosage forms may also be stamped, painted, coated, printed, etc. prior to being sealed in the blisters. These dosage forms may be stored in bulk, in blister packs, in conventional openable and reclosable multi-tablet bottles or other similar packaging.

The present invention is a delivery vehicle contemplated to be used to administer any API. It is especially useful for persons who have difficulty swallowing, geriatric persons, children, persons with disabilities, etc. The dosage form may be placed in the mouth, allowing the patient's saliva to disintegrate/dissolve the dosage form into an organoleptically pleasant slurry/solution/suspension to be easily swallowed.

The frequency of dosing depends on various factors including the amount of API present in the dosage form, the size of the dosage form, the weight of the patient, the condition of the patient, side effects of the API, etc. The administration of multiple dosage forms and multiple frequency of dosing is contemplated depending upon the above factors as well as duration of the patient's condition, how long the active ingredient stays in a patient's system etc. It is also contemplated that at least one dosage form in accordance with the present invention will be administered to a patient at least once in month time period. Such administration would include on factors given above as well as the patient's condition, the age and size of the patient, the amount of time the active ingredient stays in a patient's system, the type and kind of side effects, etc.

### EXAMPLES

### Example 1

Famotidine Protected Particles

| Materials | % |
|---|---|
| Famotidine | 37.3 |
| Sugar Spheres, | 31.2 |
| Eudragit E-100 | 16.8 |
| Magnesium Stearate | 8.3 |
| Hypromellose 2910 | 6.4 |

A 17.5% w/w aqueous dispersion of Famotidine and Hypromellose 2910 (5.9:1) was sprayed onto 60/80 sugar spheres fluidized in a fluid bed with the Wurster insert to a target potency of 49.8%. The famotidine loaded beads were then fluidized in a fluid bed with the Wurster insert onto which a 25% w/w alcoholic dispersion of Eudragit E and magnesium stearatre (2:1) was sprayed to a target potency of 37.3%.

Example 2

Blends of matrix materials illustrated below were lyophilized to produce 20mg and 40mg Famotidine tablets. Primarily, Mannitol and Lactose were used as fillers, and Dextran 70 and PVP K30 were used as binders. Sucralose was used as a sweetener.

Once blended, the matrix and protected particles of Example 1 were frozen at -40°C. Over a period of 4 hours the temperature was raised from -40°C to +40°C, during which time sublimation occurred. With non-optimized parameters, the lyophilization cycle was about 7 hours.

### Famotidine 20 mg

| Batch nbr | 023.05/1 | 023.05/2 | 024.05 | 027.05/1 | 027.05/2 | 028.05/1 | 028.05/2 |
|---|---|---|---|---|---|---|---|
| Batch size | 50 units | 50 units | 100 units | 200 units | 200 units | 200 units | 200 units |
| Components | mg | mg | mg | Mg | mg | mg | mg |
| Famotidine Protected particles | 53.62 | 53.62 | 53.62 | 53.62 | 53.62 | 53.62 | 53.62 |
| Dextran 70 | 5 | 10 | 0 | 5 | 10 | 0 | 0 |
| PVP K30 | 0 | 0 | 5 | 0 | 0 | 5 | 10 |
| Sucralose | 1 | 1 | 1 | 1 | 1 | 1 | 1 |
| Mannitol | 190.38 | 235.38 | 0 | 190.38 | 235.38 | 190.38 | 185.38 |
| Lactose | 0 | 0 | 190.38 | 0 | 0 | 0 | 0 |
| Purified water | 150 | 200 | 150 | 150 | 200 | 150 | 150 |
| Unit weight after lyophilization | 250 | 300 | 250 | 250 | 300 | 250 | 250 |
| Hardness (N) | 16.6 | 21.6 | 12.6 | 16.6 | 30.3 | 21.7 | 23.8 |
| Disintegration time (seconds) | 2 | 4 | 5 | Not measured | Not measured | 3 | 4 |

### Famotidine 40 mg

| Batch nbr | 037.05/1 | 037.05/2 | 038.05/1 | 038.05/2 |
|---|---|---|---|---|
| Batch size (units) | 50 | 50 | 150 | 150 |
| Components | mg | mg | mg | mg |
| Famotidine Protected particles | 107.24 | 107.24 | 107.24 | 107.24 |
| Dextran 70 | 10 | 10 | 0 | 0 |
| PVP K30 | 0 | 0 | 5 | 10 |
| Sucralose | 1 | 2 | 1 | 1 |
| Mannitol | 181.76 | 180.76 | 186.76 | 181.76 |
| Purified water | 200 | 200 | 200 | 200 |
| Unit weight after lyophilization | 300 | 300 | 300 | 300 |
| Hardness (N) | 30.6 | 43.3 | 19.9 | 22.1 |
| Disintegration time (seconds) | Not measured | Not measured | 4 | 3 |

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A dosage form comprising: at least one protected particle comprising an active pharmaceutical ingredient coated with a lyophilizing solvent protective coating and a matrix, said dosage form being a lyophilized, orally dissolvable/ disintegrable dosage form adapted for direct oral dosing in the mouth of a patient.

2. The dosage form of claim 1 further comprising a coating disposed under said lyophilizing solvent protective coating.

3. The dosage form of claim 2 wherein said coating disposed under said lyophilizing solvent protective coating provides taste masking for said particle, or controlled release or extended release of said active pharmaceutical ingredient of said particle.

4. The dosage form of claim 1 wherein said lyophilizing solvent protective coating provides taste masking for said particle or controlled release or extended release of said active pharmaceutical ingredient of said particle.

5. The dosage form of claim 1 wherein said particle is a grain, microparticle, crystal, granule, microgranule, agglomerate, pellets, mini- tablet, bead, microcrystal or powder.

6. The dosage form of claim 5 wherein said particle further comprises at least one excipient.

7. The dosage form of claim 6 wherein said excipient is a binder, pH adjusting substance, filler, disintegrant, solid support or buffer.

8. The dosage form of claim 1 wherein said lyophilizing solvent protective coating will dissolve at basic pH.

9. The dosage form of claim 1 wherein said lyophilizing solvent protective coating will dissolve at neutral pH.

10. The dosage form of claim 1 wherein said lyophilizing solvent protective coating will dissolve at acidic pH.

11. The dosage form of claim 10 wherein said lyophilizing solvent protective coating will dissolve at a pH of about 6.5 or less.

12. The dosage form of claim 1 wherein said lyophilizing solvent protective coating is provided in an amount of about 0.1 % to about 500% based on the weight gain of said particles.

13. The dosage form of claim 12 wherein said lyophilizing solvent protective coating is provided in an amount of about 1 % to about 200% based on the weight gain of said particles.

14. The dosage form of claim 12 wherein said lyophilizing solvent protective coating is provided in an amount of about 1% to about 100% based on the weight gain of said particles.

15. The dosage form of claim 1 wherein said matrix comprises at least one binder, lyophilization binder, filler, sugar, artificial sweetener, polymer, flavoring agent, taste masking material, active ingredient, coloring agent, lubricant, effervescent disintegrant, noneffervescent disintegrant, viscosity modifier, surfactant and buffer.

16. The dosage form of claim 15 wherein said lyophilizing solvent protective coating provides taste masking for said particle or controlled release or extended release of said active pharmaceutical ingredient of said particle, said particle is a grain, microparticle, crystal, granule, microgranule, agglomerate, pellets, mini- tablet, bead, microcrystal or powder and said lyophilizing solvent protective coating will dissolve at a pH of about 6.5 or less.

17. The dosage form of claim 1 wherein said active pharmaceutical ingredient is modafinil, famotidine or fentanyl and/or salts thereof.

18. The dosage form of claim 1, wherein said active pharmaceutical ingredient is solvent insoluble.

19. A method of making a dosage form comprising the steps of:
providing at least one active pharmaceutically ingredient containing particle, coating said particle with at least one lyophilizing solvent protective coating;
forming a mixture of said lyophilizing solvent protective coated particles with a lyophilizing solvent which will not dissolve said lyophilizing solvent protective coating and a matrix; and
lyophilizing said mixture to form a dosage form.

20. The method of claim 19 further comprising the step of placing said mixture into a portion of a container prior to lyophilzation and sealing said mixture into said dosage form after lyophilization.

21. A method of treating a patient comprising the steps of:
placing an orally dissolvable/disintegrable lyophilized dosage of claim 1 in the mouth of a patient in need of treatment;
allowing said dosage form to disintegrate/dissolve sufficiently to allow said the lyophilizing solvent protective coated particles to be swallowed as a solution, suspension or slurry; and
swallowing the at least partially disintegrated/dissolved dosage form.
